(19) Europäisches Patentamt
European Patent Office
Office européen des brevets

(11) EP 1 506 394 B1

(12) # FASCICULE DE BREVET EUROPEEN

(45) Date de publication et mention de la délivrance du brevet:
**19.07.2006 Bulletin 2006/29**

(21) Numéro de dépôt: **03752791.8**

(22) Date de dépôt: **07.05.2003**

(51) Int Cl.:
***G01N 33/38*** *(2006.01)* ***G01B 13/08*** *(2006.01)*
***G01N 15/08*** *(2006.01)*

(86) Numéro de dépôt international:
**PCT/FR2003/001419**

(87) Numéro de publication internationale:
**WO 2003/098209 (27.11.2003 Gazette 2003/48)**

(54) **DISPOSITIF DE DETERMINATION DE LA FINESSE DE FIBRES MINERALES**

VORRICHTUNG ZUR BESTIMMUNG DER FEINHEIT VON MINERALFASERN

DEVICE FOR DETERMINING FINENESS OF MINERAL FIBERS

(84) Etats contractants désignés:
**AT BE BG CH CY CZ DE DK EE ES FI FR GB GR HU IE IT LI LU MC NL PT RO SE SI SK TR**

(30) Priorité: **22.05.2002 FR 0206252**

(43) Date de publication de la demande:
**16.02.2005 Bulletin 2005/07**

(73) Titulaire: **SAINT-GOBAIN ISOVER**
**92400 Courbevoie (FR)**

(72) Inventeurs:
- **SEGHERS, Alex**
**F-60600 Fitz James (FR)**
- **BOYER, Gilles**
**F-40260 Lesperon (FR)**

(74) Mandataire: **Jamet, Vincent**
**Saint-Gobain Recherche,**
**39, quai Lucien Lefranc**
**93300 Aubervilliers (FR)**

(56) Documents cités:
**EP-A- 0 373 058** **US-A- 4 396 943**
**US-A- 4 440 021** **US-A- 5 359 880**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).



Printed by Jouve, 75001 PARIS (FR)

## Description

La présente invention est relative à un dispositif de détermination de la finesse de fibres minérales, ces fibres étant destinées notamment à la fabrication industrielle de laine de verre devant entrer par exemple dans la composition de produits d'isolation thermique et/ou acoustique. Elle vise également un procédé de mesure de la finesse de fibres minérales ainsi que l'application de ce dispositif à la mesure de finesse de fibres hyper fines.

Elle vise plus précisément à permettre la détermination de la valeur de leur « micronaire » (F).

La mesure du « micronaire » est connue dans ce domaine technique pour caractériser l'indice de finesse, cet indice rendant compte de la surface spécifique en fonction de la mesure de la perte de charge aérodynamique lorsqu'une quantité donnée de fibres, dépourvues de liant, est soumise à une pression donnée d'un gaz - en général de l'air ou de l'azote.

Cette mesure est usuelle dans les unités de production de fibres naturelles (notamment des fibres de coton) et est normalisée selon les normes DIN 53941, ASTM, 1994, D 4604-86, D 4605-86 ou encore EN 29053 et elle utilise un appareil dit « appareil micronaire » comme est décrit dans le document EP 0 373 058.

De plus, on note que généralement la masse de l'échantillon est de l'ordre de 10 g et peut atteindre selon les préconisations les plus couramment employées 50 g, lorsqu'il s'agit de fibres de coton.

Sur la base de ces enseignements, on a développé et on utilise couramment un appareil micronaire, qui indique, à partir d'un dispositif de mesurage de l'indice de finesse de fibres minérales, gradué en valeur « micronaire », des mesures fiables pour des diamètres moyens de fibres compris sensiblement entre 3 et 9 μm et dans cette gamme de diamètre moyen. Ce diamètre moyen est calculé à partir de l'histogramme mesuré au microscope (x 1000) de 200 fibres.

De plus en plus, les fabricants, dans leur recherche constante d'amélioration du coefficient d'échange thermique global et/ou de la diminution de la masse volumique des produits pour une même capacité thermique, sont amenés à élaborer des fibres (nommées fibres hyper fines dans la suite du texte) dont le diamètre moyen est en constante diminution et tend à être compris dans la fourchette 2 à 3 μm, voire beaucoup moins.

Or, dans ces gammes de fabrications de fibres hyper fines, la mesure « micronaire » décrite ci-dessus n'est pas possible.

Ces fabrications de fibres hyper fines nécessitent cependant un instrument de mesure permettant de suivre, quasiment en temps réel, le processus de fabrication afin de pouvoir rapidement intervenir sur le process en cas de dysfonctionnement.

Dans un souci de rationalisation, on a posé comme hypothèse de conserver le principe de l'appareil micronaire traditionnel, cet appareil ayant prouvé son efficacité pour des fibres minérales dont le diamètre est supérieur à 3 μm et dont les avantages sont appréciés par les professionnels du domaine technique considéré (mise en oeuvre aisée, grande simplicité, fiabilité, rapidité de mise en oeuvre, économique... ).

Partant de ce constat et connaissant l'inaptitude de cet appareil micronaire à fournir des mesures pour des fibres hyper fines, la présente invention se propose de fournir un appareil micronaire adapté pour fournir des valeurs micronaire pour des fibres hyper fines (dont le diamètre moyen est inférieur à 3 μm).

On connaît un dispositif de détermination de l'indice de finesse de fibres adapté pour fournir la valeur micronaire de fibres minérales dont le diamètre moyen est sensiblement supérieur à 3 μm comportant un dispositif de mesurage de l'indice de finesse, ledit dispositif de mesurage de l'indice de finesse étant pourvu d'une part, d'au moins un premier orifice relié à une cellule de mesure adaptée pour recevoir un échantillon constitué d'une pluralité de fibres, et d'autre part, d'un second orifice relié à un dispositif de mesurage d'une pression différentielle située de part et d'autre dudit échantillon, ledit dispositif de mesurage de la pression différentielle étant destiné à être relié à un dispositif de production d'écoulement de fluide.

A cet effet, selon l'invention, un dispositif et un procédé de détermination de l'indice de finesse du genre en question est caractérisé en ce que le dispositif de mesurage de l'indice de finesse comporte au moins un débitmètre volumétrique du gaz traversant ladite cellule, selon les revendications 1 et 5.

Dans des modes de réalisation préférés de l'invention, on peut éventuellement avoir recours en outre à l'une et/ou à l'autre des dispositions suivantes :

- le débitmètre volumétrique est constitué par un débimètre volumétrique gradué en l/mn ;
- l'un des orifices comporte un organe calibré ;

Selon un autre aspect de l'invention, celle-ci vise également un procédé de mesure de la finesse de fibres utilisant un dispositif de détermination de la finesse de fibres minérales tel que visé précédemment caractérisé en ce que :

- on remplit la cellule de mesure d'un échantillon de fibres, la masse d'échantillon étant déterminée de telle façon que l'échantillon occupe tout le volume de ladite cellule de mesure, afin qu'il n'y ait pas d'écoulement préférentiel de gaz au sein de l'échantillon,
- on règle la valeur de la pression différentielle régnant entre l'amont et l'aval de l'échantillon après avoir refermé ladite cellule à l'aide d'un couvercle,
- on relie ledit dispositif de mesure à un dispositif de production d'écoulement de fluide,
- on procède à la mesure à l'aide du débitmètre volumétrique.

Dans des modes de réalisation préférés de l'invention, on peut éventuellement avoir recours en outre à l'une et/ou à l'autre des dispositions suivantes :

- on remplit la totalité du volume de la cellule de mesure avec un échantillon de fibres dont la masse est égale à au moins 5 g, de préférence comprise entre 5 à 10 g, et encore plus préférentiellement égale à 5 g
- on applique une pression différentielle dont la valeur est sensiblement voisine de 254 mm Hg.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue schématique d'un appareil « micronaire » selon l'invention;
- la figure 2 donne pour diverses masses volumiques de fibres, la valeur du coefficient de conductibilité thermique en fonction de la valeur micronaire exprimée en l/mn,

Sur les différentes figures, les mêmes références désignent des éléments identiques ou similaires.

Sur la figure 1, on a représenté un appareil micronaire selon l'invention. Cet appareil 1 comporte une cellule de mesure 2, préférentiellement cylindrique. Cette cellule 2 est adaptée pour recevoir un échantillon de fibres, pour lesquelles on recherche la mesure de leur finesse

Cet appareil comporte également un dispositif de mesurage 8 de l'indice de finesse.

Ce dispositif de mesurage 8 de l'indice de finesse comporte au moins un premier orifice 3 relié par l'intermédiaire d'une première conduite 4 à la cellule de mesure 2 adaptée pour recevoir l'échantillon constitué d'une pluralité de fibres.

Ce même dispositif de mesurage 8 de l'indice de finesse comporte par ailleurs un second orifice 6 relié par l'intermédiaire d'une seconde conduite 7 à un dispositif de mesurage 5 d'une pression différentielle située de part et d'autre dudit échantillon. Ce dispositif de mesurage 5 de la pression différentielle est constitué par exemple par un régulateur de pression dont la valeur est fixée et est maintenue constante à 254 mm de Hg.

Dans un appareil « micronaire » traditionnel (c'est-à-dire adapté pour indiquer des valeurs « micronaire » pour des fibres dont le diamètre moyen est compris dans la gamme 3 à 9 μm), le dispositif de mesurage 8 de l'indice de finesse est constitué par un tube cylindrique transparent de section droite circulaire, au sein duquel peut se translater un indicateur mobile (du type ludion), ce tube étant gradué en valeur « micronaire ».

Le dispositif de mesurage 5 de la pression différentielle est relié par une troisième conduite 9 à un dispositif de production 10 d'écoulement de gaz constant, et le gaz pouvant être classiquement de l'air ou de l'azote. Quelle que soit la source d'écoulement de gaz utilisée, l'installation doit permettre un réglage précis du débit et un contrôle de stabilité de l'écoulement de gaz dans la partie inférieure de la cellule de mesure.

La source de l'écoulement de fluide doit délivrer le fluide à des débits tels que les vitesses qui en résultent soient suffisamment faibles pour que les résistances à l'écoulement du fluide mesurées soient indépendantes de la vitesse.

A titre d'exemple, la source d'écoulement doit permettre d'obtenir des vitesses d'écoulement de fluide pouvant atteindre $0{,}5.10^{-3}$ m/s.

En variante, l'appareil « micronaire » représenté en figure 1, comporte au niveau de l'orifice 6, une buse calibrée. Cet organe calibré, et notamment le diamètre de l'orifices de la buse, est déterminé lors de l'étalonnage de l'appareil « micronaire », dans des conditions normales de pression et de température (T=20°C, P= 101325 Pa).

Le principe de la mesure de l'indice de mesure de la finesse (ou valeur micronaire) est basé sur une mesure de perméabilité d'un milieu poreux, homogène et isotrope, ce milieu poreux (en l'occurrence un échantillon de fibres dont on veut connaître le diamètre moyen) étant traversé par un fluide gazeux, dans des conditions d'écoulement laminaire.

Le principe de la mesure est régi par la loi de Darcy :

$$v = \frac{Q}{S} = \frac{K}{\mu} \times \frac{\Delta P}{e} \quad (1)$$

v : vitesse du fluide (m/s)
Q : débit du fluide ($m^3/s$)
S : section de passage de l'échantillon traversée perpendiculairement par le fluide ($m^2$)
ΔP : pression différentielle au travers de l'échantillon ($N/m^2$)
e : épaisseur de l'échantillon (m)
μ : viscosité dynamique du fluide (N/m.s)
K : perméabilité ($m^2$)

Des études menées par KOZENY et CARMAN ont montré que le coefficient K pouvait s'exprimer de la manière suivante :

$$K = \frac{1}{j} \times \frac{\varepsilon^3}{(1-\varepsilon)^2} \times \frac{1}{(Sv)^2} \quad (2)$$

où

K : perméabilité
j : facteur de structure

$$\varepsilon : \text{porosité du milieu} = 1 - \frac{m}{\rho \times S \times e}$$

Sv : surface spécifique
M : masse de l'échantillon
p : densité de l'échantillon

En combinant les équations (1) et (2), et en posant constantes les grandeurs suivantes (densité, porosité, facteur de structure, quantité d'échantillon introduit), le débit Q varie d'une manière inversement proportionnelle au carré de la surface spécifique selon la relation suivante

$$Q = \frac{1}{(Sv)^2}$$

Le mode opératoire de l'appareil « micronaire » selon l'invention est le suivant.

A partir d'un dispositif de détermination de la finesse de fibres de l'art antérieur (c'est-à-dire adapté pour délivrer des valeurs « micronaire » pour des fibres dont le diamètre moyen est sensiblement compris dans la gamme 3 à 9 μm), et après avoir étalonné ce dernier par rapport à un appareil de référence avec un échantillon de fibres de référence, de manière à obtenir la courbe visée en figure 2, on remplit la cellule 2 de mesure d'au moins 5g de fibres dont on veut déterminer la finesse (ces fibres étant vierges, c'est-à-dire dépourvues de liant).

Il convient de remarquer que la quantité de fibres introduite dans la cellule de mesure est déterminante. On a noté qu'avec une quantité de fibres inférieure à 5g, (par exemple 3g), surtout lorsque ces fibres sont hyper fines, celles-ci n'occupent pas tout le volume de la cellule de mesure, et il se crée des écoulements préférentiels au sein de la cellule qui affectent singulièrement la mesure. Ainsi, on a pu constater une non reproductibilité des résultats de la mesure (variation de plus de 50 % du résultat).

En pratique, expérimentalement on a fait des essais avec des différentes quantités de fibres, et on a déterminé que la quantité optimale permettant d'obtenir une reproductibilité satisfaisante de la mesure consistait à prendre au moins 5 g de fibres.

On remplit la cellule de mesure 2 de 5g de fibres vierges, la mesure de la masse de fibres étant réalisée à l'aide d'un appareil de précision, (la masse de fibres se situant en fait dans l'intervalle 5.00 +/- 0.01 g), cette masse de fibres ayant été prélevée à l'aide d'une pelle, juste après l'outil de fibrage, et avant le dépôt du liant

La cellule de mesure 2 est une chambre cylindrique dont les dimensions caractéristiques sont les suivantes Diamètre intérieur : 25,4mm ; hauteur : 25,4mm, dont l'ouverture est obturée par un couvercle 13.

L'étape suivante consiste dans le réglage de la pression différentielle statique régnant entre l'amont et l'aval de l'échantillon de fibres, par hypothèse, cette différence de pression est fixée à 254 mm de Hg.

On relie l'appareil de détermination de la finesse selon l'invention au dispositif de production d'écoulement de gaz et on procède à la mesure à l'aide de l'indicateur du débitmètre volumétrique.

Sur la figure 2, on a porté pour diverses fabrications de fibres, correspondant à une gamme de trois familles de produits bien définis, chacune de ces familles étant caractérisée par la valeur de son coefficient de conductibilité thermique (λ), l'évolution de la conductibilité thermique pour des diverses valeurs du micronaire, obtenue par le dispositif objet de l'invention,

Ainsi, on définit :

- les fibres pour produits légers enroulés (ou IBR) ayant une masse volumique MV comprise entre 10-11 kg/m$^3$,
- les fibres pour les produits roulés denses ou pour panneaux ou cloisons légers, ayant une masse volumique comprise entre 15-16 kg/m$^3$,
- et enfin les fibres pour panneaux denses ayant une masse volumique comprise entre 22-23 kg/m$^3$,

A titre indicatif, on peut noter une relation de correspondance entre les valeurs micronaire ainsi obtenue et la valeur du diamètre moyen de l'échantillon de fibres. Globalement, une valeur micronaire d'environ 12 l/mn correspond à un diamètre moyen de 2.5 à 3 μm, une valeur de 13.5 l/mn correspond sensiblement à un diamètre moyen de 3 à 3.5 μm, et enfin 18 l/mn à environ 4 à 5 μm. On rappelle que pour ces trois gammes de produit, la valeur micronaire (obtenue par un appareil traditionnel) n'est pas possible pour un diamètre moyen de 2.5 à 3 μm, elle vaut sensiblement 2.7 pour un diamètre moyen de 3 à 3.5 μm, et enfin 3 à environ 4 à 5 μm

Bien entendu, en fonction de la gamme de fibres dont on veut caractériser le diamètre moyen, il est possible d'adapter le dispositif de mesurage de la finesse (gamme du débitmètre volumétrique) afin d'obtenir pour toutes les productions souhaitées de fibres, une même unité de mesure (l/mn).

Ainsi, pour des fibres encore plus fines (diamètre moyen de l'ordre de sensiblement 1 μm, voire inférieure), il a été possible avec le dispositif objet de l'invention d'obtenir une valeur reproductible et fiable, à savoir de l'ordre de 1l/min.

## Revendications

1. Dispositif de détermination de l'indice de finesse ou valeur « micronaire » de fibres (1) minérales, de diamètre moyen inférieur à 3 μm, ledit dispositif (1) com-

portant un dispositif de mesurage (8) de l'indice de finesse, ledit dispositif de mesurage (8) de l'indice de finesse étant pourvu d'une part, d'au moins un premier orifice (3) relié à une cellule de mesure (2) adaptée pour recevoir un échantillon constitué d'une pluralité de fibres et d'autre part, d'un second orifice (6) relié à un dispositif de mesurage (5) d'une pression différentielle située de part et d'autre dudit échantillon, la pression différentielle entre l'amont et l'aval de l'échantillon étant réglée après avoir refermé ladite cellule, ledit dispositif de mesurage (5) de la pression différentielle étant destiné à être relié à un dispositif de production d'écoulement de fluide (10), **caractérisé en ce que** le dispositif de mesurage (8) de l'indice de finesse comporte au moins un débitmètre volumétrique du fluide traversant ladite cellule (2).

2. Dispositif selon la revendication 1, **caractérisé en ce que** le débitmètre volumétrique est gradué en l/mn.

3. Dispositif selon l'une des revendications 1 ou 2, **caractérisé en ce que**' orifice (6) comporte un organe calibré (12).

4. Dispositif selon l'une des revendications 1 à 3, **caractérisé en ce que** le dispositif de mesurage (8) de l'indice de finesse est adapté en fonction de la gamme de fibres dont on veut caractériser le diamètre moyen.

5. Procédé de mesure de la finesse de fibres minérales utilisant un dispositif selon l'une des revendications 1 à 4, **caractérisé en ce que :**

   - on remplit la cellule de mesure (2) d'un échantillon de fibres dont le diamètre moyen est inférieur à 3μm, la masse d'échantillon étant déterminée de telle façon que l'échantillon occupe tout le volume de ladite cellule de mesure (2), afin qu'il n'y ait pas d'écoulement préférentiel de gaz au sein de l'échantillon,
   - on règle la valeur de la pression différentielle régnant entre l'amont et l'aval de l'échantillon après avoir refermé ladite cellule à l'aide d'un couvercle (13),
   - on relie ledit dispositif de mesure à un dispositif de production d'écoulement de fluide (10),
   - on procède à la mesure à l'aide du débitmètre volumétrique.

6. Procédé selon la revendication 5, **caractérisé en ce qu'**on remplit la totalité du volume de la cellule de mesure (2) avec un échantillon de fibres dont la masse est égale à 5 g et, de préférence comprise entre 5 à 10 g, et encore plus préférentiellement égale à 5 g

7. Procédé selon l'une des revendications 5 ou 6, **caractérisé en ce qu'on applique** une pression différentielle dont la valeur est sensiblement voisine de 254 mm Hg.

8. Application du dispositif de mesure selon l'une des revendications 1 à 4 et ou du procédé selon les revendications 5 à 7, à la mesure de l'indice de finesse pour des fibres fines d'isolation, notamment de laine de verre, notamment obtenues par un procédé de centrifugation interne.

## Claims

1. Apparatus (1) for determining the fineness index of mineral fibres, designed to deliver the "micronaire" value of fibres, with a mean diameter inferior to 3 μm, the said apparatus (1) comprising a device (8) for measuring the fineness index, the said device (8) for measuring the fineness index being provided, on the one hand, with at least a first orifice (3) connected to a measurement cell (2) designed to hold a specimen consisting of a plurality of fibres and, on the other hand, with a second orifice (6) connected to a device (5) for measuring a differential pressure, on either side of the said specimen, the said value of the differential pressure between the upstream end and the downstream end of the specimen being adjusted after the said cell has been closed, the said device (5) for measuring the differential pressure being intended to be connected to a device (10) for producing a fluid flow, **characterized in that** the device (8) for measuring the fineness index includes at least one flowmeter for measuring the volume flow rate of the fluid passing through the said cell (2).

2. Apparatus according to Claim 1, **characterized in that** the volume flowmeter is graduated in l/min.

3. Apparatus according to either of Claims 1 and 2, **characterized in that** the orifice (6) includes a calibrated member (12).

4. Apparatus according to one of Claims 1 to 3, **characterized in that** the device (8) for measuring the fineness index is adapted according to the range of fibres whose mean diameter it is desired to characterize.

5. Method of measuring the fineness of mineral fibres using an apparatus according to one of claims 1 to 4, **characterized in that**:

   - the measurement cell (2) is filled with a specimen of fibres with a mean diameter inferior to 3 μm, the specimen mass being determined in such a way that the specimen occupies the entire volume of the said measurement cell (2) so that there is no preferential flow of gas within the specimen;
- the value of the differential pressure between the upstream end and the downstream end of the specimen is adjusted after the said cell has been closed using a lid (13);
- the said measurement apparatus is connected to a device for producing a flow of fluid (10); and
- the measurement is taken using the volume flowmeter.

**6.** Method according to Claim 5, **characterized in that** the entire volume of the measurement cell (2) is filled with a specimen of fibres, the mass of which is equal to 5 g, preferably between 5 and 10 g, and even more preferably equal to 5 g.

**7.** Method according to either of Claims 5 and 6, **characterized in that** a differential pressure, the value of which is approximately 254 mmHg, is applied.

**8.** Application of the measurement apparatus according to one of Claims 1 to 4 and/or of the method according to Claims 5 to 7 to the measurement of the fineness index for fine insulation fibres, especially glass wool, particularly those obtained by an internal centrifuging process.

## Patentansprüche

**1.** Vorrichtung zur Bestimmung des Feinheitsindexes oder Micronairewerts von Mineralfasern (1), deren mittlerer Durchmesser unterhalb 3 μm liegt, wobei die Vorrichtung (1) eine Messvorrichtung (8) für den Feinheitsindex aufweist, wobei die Messvorrichtung (8) für den Feinheitsindex auf der einen Seite mit wenigstens einer ersten Öffnung (3) versehen ist, die mit einer Messzelle (2) verbunden ist, welche geeignet ist eine Probe bestehend aus einer Vielzahl von Fasern aufzunehmen und auf der anderen Seite mit einer zweiten Öffnung (6) versehen ist, verbunden mit einer Messvorrichtung (5) zur Messung des Differenzdrucks, die auf der einen und auf der anderen Seite der genannten Probe angeordnet ist, wobei der Differenzdruck zwischen Zulauf und Ablauf der Probe nach Schließen der Zelle gesteuert wird und wobei die Messvorrichtung (5) zur Messung des Differenzdrucks dazu bestimmt ist mit einer Vorrichtung zum Erzeugen einer Flüssigkeitsströmung (10) verbunden zu werden, **dadurch gekennzeichnet, dass** die Vorrichtung (8) zur Messung des Feinheitsindexes wenigstens einen Volumendurchflussmesser für Flüssigkeit, die die genannte Zelle (2) durchfließt, umfasst.

**2.** Vorrichtng nach Anspruch 1, **dadurch gekennzeichnet, dass** der Volumendurchflussmesser auf 1/min geeicht ist.

**3.** Vorrichtung nach Anspruch 1 oder 2, **dadurch gekennzeichnet, dass** die Öffnung (6) ein kalibriertes Element (12) umfasst.

**4.** Vorrichtung nach einem der Ansprüche 1 bis 3, **dadurch gekennzeichnet, dass** die Messvorrichtung (8) des Feinheitsindexes entsprechend dem Bereich der Fasern angepasst ist, deren mittlerer Durchmesser bestimmt werden soll.

**5.** Verfahren zur Bestimmung des Feinheitsindexes von Mineralfasern unter Verwendung einer Vorrichtung nach einem der Ansprüche 1 bis 4, **dadurch gekennzeichnet, dass**:

- die Messzelle (2) mit einer Probe von Fasern befüllt wird, deren mittlerer Durchmesser unterhalb von 3 μm liegt, wobei die Masse der Probe derart bestimmt wurde, dass die Probe das gesamte Volumen der Messzelle (2) einnimmt, damit es keine bevorzugte Strömung des Gases inmitten der Probe mehr gibt,
- der Wert des Differenzdrucks, der zwischen dem Zulauf und Ablauf der Probe herrscht, gesteuert wird, nachdem man die Zelle mit Hilfe eines Dekkels (13) verschlossen hat,
- die Messvorrichtung mit einer Vorrichtung zum Erzeugen einer Flüssigkeitsströmung (10) verbunden wird,
- die Messung mit Hilfe eines Volumendurchflussmessers durchgeführt wird.

**6.** Verfahren nach Anspruch 5, **dadurch gekennzeichnet, dass** die Gesamtheit des Volumens der Messzelle (2) mit einer Probe von Fasern befüllt wird, deren Masse gleich 5 g ist, bevorzugt zwischen 5 und 10 g liegt, stärker bevorzugt gleich 5 g ist.

**7.** Verfahren nach einem der Ansprüche 5 und 6, **dadurch gekennzeichnet, dass** ein Differenzdruck angelegt wird, dessen Wert deutlich an 254 mm Hg angrenzt.

**8.** Verwendung einer Messvorrichtung nach einem der Ansprüche 1 bis 4 und/oder eines Verfahrens nach einem der Ansprüche 5 bis 7, zur Messung des Feinheitsindexes für feine Dämmfasern, besonders Glaswolle, insbesondere Fasern, die durch ein Verfahren der internen Zentrifugation erhalten wurden.

Figure 1

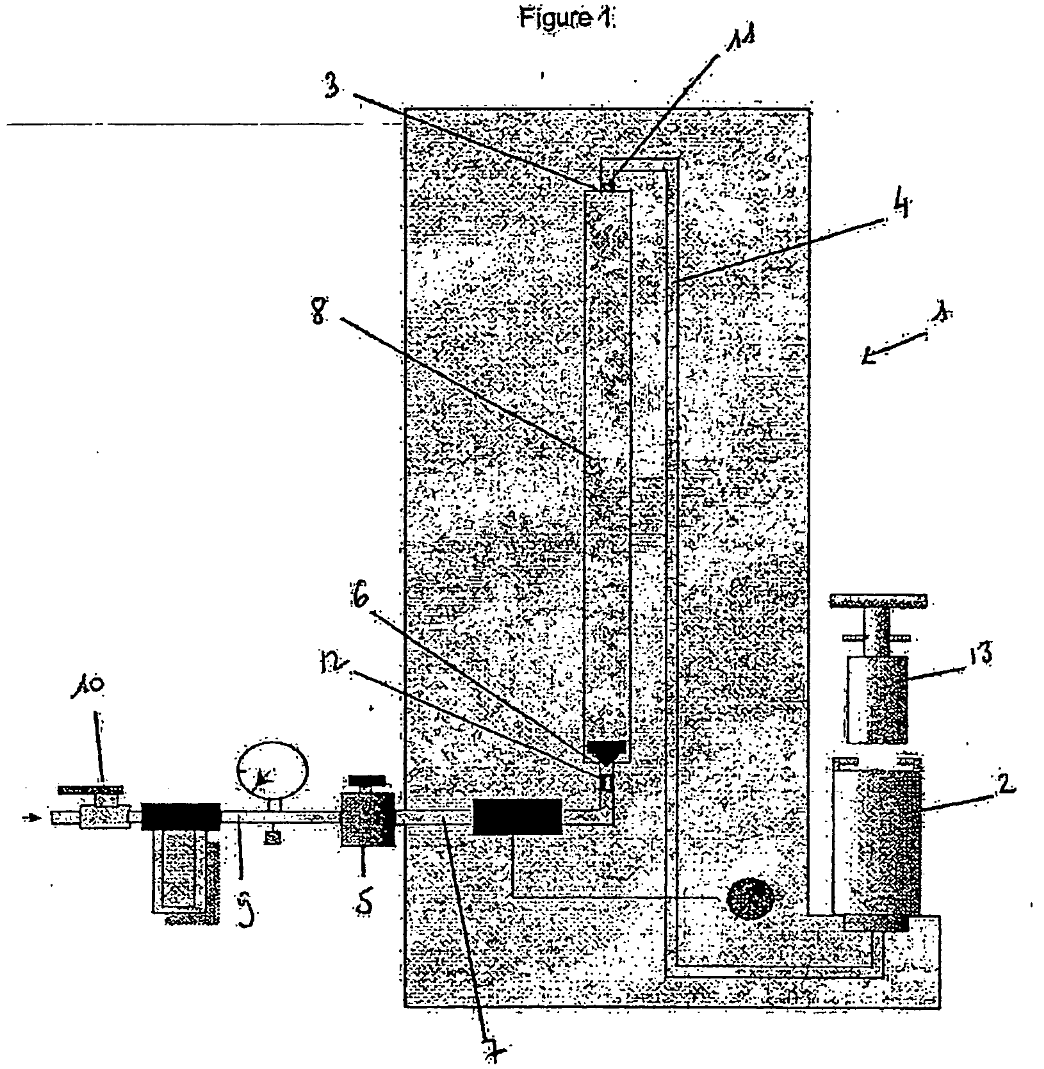

Figure 2

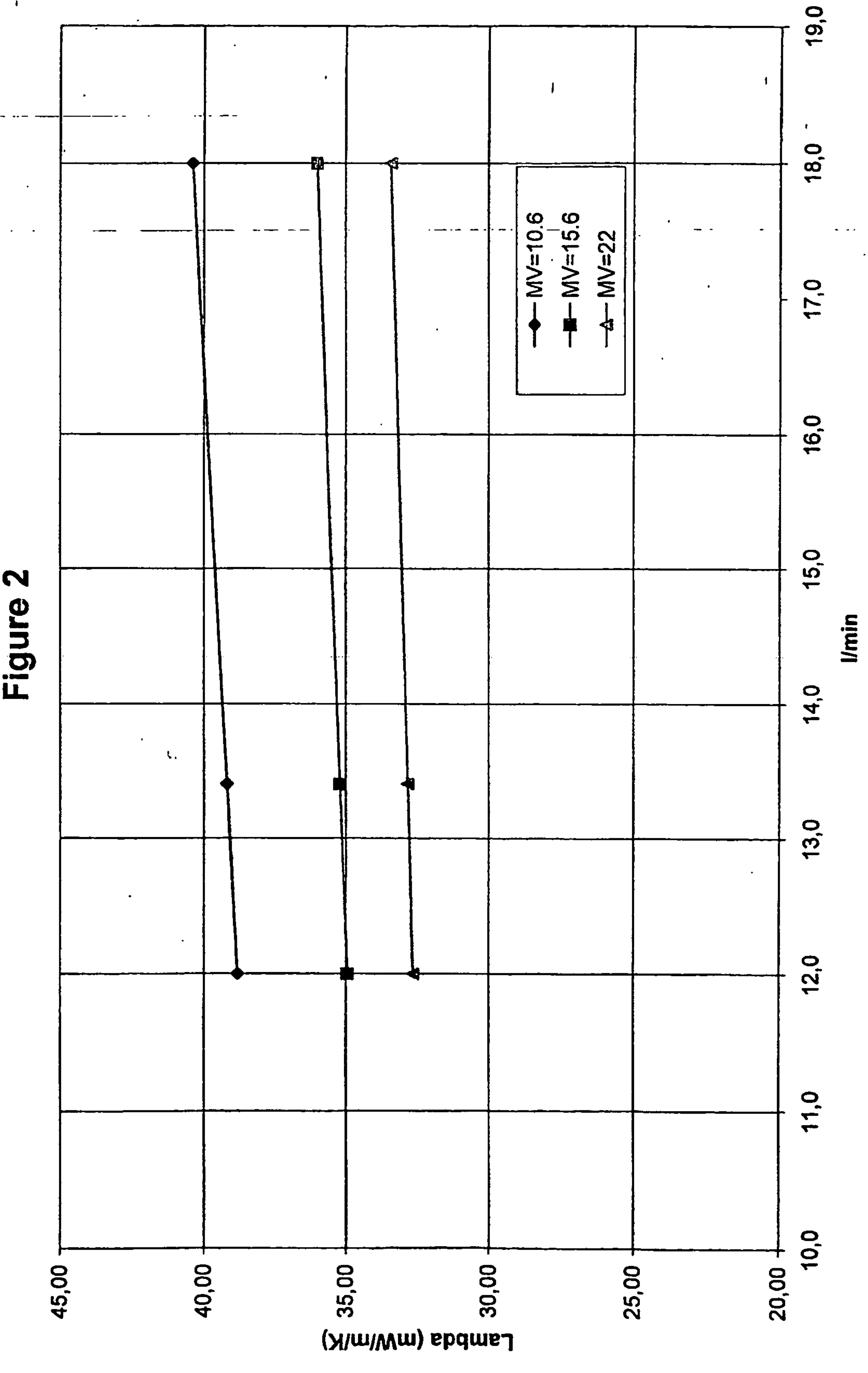
MV=10.6
MV=15.6
MV=22
l/min
Lambda (mW/m/K)
45,00
40,00
35,00
30,00
25,00
20,00
10,0
11,0
12,0
13,0
14,0
15,0
16,0
17,0
18,0
19,0